Europäisches Patentamt

European Patent Office

Office européen des brevets

(19)

(11) **EP 1 372 601 B1**

(12) **EUROPÄISCHE PATENTSCHRIFT**

(45) Veröffentlichungstag und Bekanntmachung des Hinweises auf die Patenterteilung:
**06.10.2004 Patentblatt 2004/41**

(21) Anmeldenummer: **02737761.3**

(22) Anmeldetag: **27.03.2002**

(51) Int Cl.$^7$: **A61K 7/48**

(86) Internationale Anmeldenummer:
**PCT/DE2002/001147**

(87) Internationale Veröffentlichungsnummer:
**WO 2002/080875 (17.10.2002 Gazette 2002/42)**

(54) **KOSMETISCHE VITAMIN-A-HALTIGE ZUBEREITUNG**

COSMETIC PREPARATION CONTAINING VITAMIN A

PREPARATION COSMETIQUE RENFERMANT DE LA VITAMINE A

(84) Benannte Vertragsstaaten:
**AT BE CH CY DE DK ES FI FR GB GR IE IT LI LU MC NL PT SE TR**

(30) Priorität: **04.04.2001 DE 10117842**

(43) Veröffentlichungstag der Anmeldung:
**02.01.2004 Patentblatt 2004/01**

(73) Patentinhaber: **Coty B.V.**
**2031 CC Haarlem (NL)**

(72) Erfinder:
 • **GOLZ-BERNER, Karin**
 **MC-98000 Monaco (MC)**

 • **ZASTROW, Leonhard**
 **MC-98000 Monaco (MC)**

(74) Vertreter: **Walter, Wolf-Jürgen et al**
**Anwaltskanzlei**
**Gulde Hengelhaupt Ziebig & Schneider**
**Wallstrasse 58/59**
**10179 Berlin (DE)**

(56) Entgegenhaltungen:
**WO-A-96/31194          WO-A-99/66881**
**US-B1- 6 183 774**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft eine Vitamin-A-haltige Zubereitung und deren Verwendung als Kosmetikum.

Der Einsatz von Vitamin A und dessen Derivaten in der Kosmetik ist seit langem bekannt. Dabei sind auch eine Reihe von Veröffentlichungen bekannt, in denen Retinol oder Retinoide mit anderen Komponenten kombiniert worden sind, um Wirksamkeit und Stabilität dieser Verbindungen zu verbessern. Die EP-0666735 B1 beschreibt die Verwendung eines Hautaufhellungsmittels, bei dem Retinole mit verschiedenen Stoffen wie Ascorbinsäure, Niacin, Kojisäure, Süßholzextrakt und anderen kombiniert werden. Eine Zusammenstellung von Retinoiden mit Vitamin B3-Verbindungen ist aus WO 00/24371 bekannt. Die WO 00/27352 beschreibt topische Zusammensetzungen mit Kollagen-verstärkender Wirkung, die ein Weizenprotein, ein Retinoid, ein Vitamin E-Derivat und Ascorbinsäure enthalten.

Die WO 99/66881 offenbart eine kosmetische Wirkstoffzubereitung mit hohem Radikalschutzfaktor, welche neben Retinylpalmitat auch Seidenraupenextrakt sowie einen Extrakt von Quebraco blanco enthält.

Die WO 96/31194 sowie US 6183774 beschreiben die Stabilisierung von Retinoiden und deren Derivate durch Verkapselung in Liposomen.

Der Erfindung liegt die Aufgabe zugrunde, die kosmetische Wirkung von Retinol bzw. Retinoiden auf der Haut deutlich zu verbessern, insbesondere zu verlängern.

Erfindungsgemäß wird diese Aufgabe durch eine Kombination verschiedener Anwendungsformen des Retinols oder seiner Derivate zusammen mit einem speziellen Pflanzenextrakt gelöst.

Die erfindungsgemäße kosmetische Vitamin-A-haltige Zubereitung besteht daher aus

a) 0,001 bis 1,0 Gew-% eines Retinylderivates, ausgewählt unter Retinylpalmitat, Retinylascorbat und Gemischen davon, bezogen auf das Gewicht der kosmetischen Zubereitung;

b) 0,01 bis 10 Gew-% eines Retinylderivates, ausgewählt unter Retinylpalmitat, Retinylascorbat und Gemischen davon und verkapselt in phospholipidhaltigen Liposomen und mit einer Teilchengröße der Liposomen im Bereich von 150 bis 800 nm, wobei der Gehalt an Retinylderivat auf das Gewicht der Liposomen bezogen ist;

c) 0,01 bis 5 Gew-% Mikrokapseln mit reinem Retinol, wobei die Teilchengröße der Kapseln von 40 bis 100 nm beträgt, und die reines Retinol im Bereich von 0,5 bis 3 Gew-% enthalten, bezogen auf das Gewicht der gefüllten Kapseln;

d) 0,1 bis 20 Gew-%, bezogen auf das Gewicht der kosmetischen Zubereitung, eines Extraktes ausgewählt unter den wäßrigen Fruchtextrakten Kirsch-Extrakt (Prunus cerasus), Acerola-Extrakt (Malpighia punicifolia), Ananas-Extrakt, hydrolysiertem Rindenextrakt von Quebracho blanco, Seidenraupenextrakt und Gemischen dieser Extrakte, mit einem Gehalt des Extraktes von wenigstens einer Retinolverbindung, ausgewählt unter Retinol, Retinylpalmitat, Retinylascorbat und Gemischen davon, im Bereich von 0,01 bis 5 Gew-%, wobei diese Angabe auf das Trockengewicht des Extraktes bezogen ist; und

e) einem Rest bis 100 Gew-% an kosmetische Hilfsstoffen, Trägerstoffen und Gemischen davon.

Ein besonders bevorzugter Kirschextrakt ist der wäßrige Extrakt der spanischen Cherry-Kirsche.

Der Kirschextrakt enthält vorzugsweise Retinylpalmitat im Gemisch zusammen mit wenigstens einem weiteren Vitamin, ausgewählt unter einem Vitamin E-Derivat, Vitamin C, Vitamin F und Gemischen davon, und Carotinoiden. Der Extrakt ist vorzugsweise verkapselt in Kapseln von 30-100 µm Durchmesser. Die Planzenextrakte sind vorzugsweise mit 3-20%, insbesondere 5-20 Gew-% enthalten.

Carotinoide im Sinne der Erfindung sind beispielsweise β-Carotin, Flavoxanthin, Neurosporin, Lycopin. Vitamin E-Derivate im Sinne der Erfindung sind beispielsweise Tocopherylacetat, Tocopherylpalmitat.

Eine weitere Ausführungsform der Erfindung besteht darin, daß der Extrakt vorzugsweise ein Extraktgemisch der spanischen Cherry-Kirsche (Prunus cerasus) und der Acerola-Frucht (Malpighia punicifolia) ist.

Es wurde überraschend gefunden, daß durch die Kombination der verschiedenen freien und verkapselten Retinol/Retinoid-Formen zusammen mit bestimmten Pflanzenextrakten, die Retinol bzw. andere Vitamine und deren Vorläufer enthalten, eine längere Bindung des Retinols an die Hautoberfläche bzw. in den oberen Hautschichten stattfindet als bei Einzelanwendung, und dadurch eine deutlich erhöhte Wirksamkeit bei gleichen oder ähnlichen Konzentrationen üblicher Retinolgehalte erreicht werden kann. Als Wirksamkeit der Retinolderivate wird insbesondere angesehen die Stimulierung der Zellumwandlung der Epidermis und die Differenzierung der neu gebildeten Keratinocyten. Dadurch tritt ein merklicher Anti-Falten-Effekt auf und ein Schutz gegen freie Radikale, der in der vorliegenden Kombination überraschend über einen sehr langen Zeitraum andauert.

Die ein Retinylderivat enthaltenden Liposomen sind vollständig geschlossene Lipid-Bilayer-Membranen auf Basis von Phospholipiden, die ein wäßriges Volumen eingeschlossen enthalten. Liposome können unilamellare Vesikel sein (die eine Einzelmembran-Bilayer besitzen) oder multilamellare Vesikel (Onionähnliche Strukturen, gekennzeichnet durch Mehrfachmembran-Bilayer, von denen jede von der nächsten durch eine wäßrige Schicht getrennt ist). Die Bilayer besteht aus zwei Lipid-Monolayern, die einen hydrophoben "Schwanz"-Bereich und einen hydrophilen "Kopf"-Bereich haben. Die Struktur der Membran-Bilayer ist so, daß die hydrophoben (unpolaren) "Schwänze" der Lipidmonolayer sich in Richtung des Zentrums der Bilayer orientieren, während sich die hydrophilen "Köpfe" in Richtung der wäßrigen Phase orientieren.

Die Herstellung von Liposomen, aus gesättigten und ungesättigten Lipiden, ist in sehr vielen Patenten beschrieben worden, ebenso deren Einsatz als Transportsystem. Die Einarbeitung Retinylderivates kann auf übliche Weise erfolgen.

Als Phospholipide können z.B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phoslipon® oder NAT®.

Die Retinol enthaltenden Mikrokapseln bestehen vorzugsweise aus einer Hülle, hergestellt aus einem Carboxymethylcellulose-Chitosan-Komplex. Diese Hülle aus einem Biopolymeren stabilisiert das in einem wäßrigen oder wäßrig-öligem Medium suspendierte Retinol gegen spontane Abbaureaktionen. Spezielle Zusätze zur Konservierung/Stabilisierung und zur Emulgierung können enthalten sein, wie z.B Parabene, Cellulosegummi, Sorbitanfettsäureester, Filmbildner usw.

Die erfindungsgemäße kosmetische Zubereitung kann neben den für die Kombinationswirkung genannten Bestandteilen weitere Wirkstoffe und kosmetische Hilfs- und Trägerstoffe enthalten.

Zu den kosmetischen Wirkstoffen gehören z. B. Emulgatoren, anorganische und organische Lichtschutzmittel, weitere Radikalfänger, Feuchthaltemittel, weitere Vitamine, Enzyme,
weitere pflanzliche Wirkstoffe, Polymere, Melanin, Antioxidationsmittel, entzündungswidrige natürliche Wirkstoffe, mit Sauerstoff beladene Fluorcarbone oder Sauerstoff tragende asymmetrische lamellare Aggregate gemäß WO 94/00109; magnetische Einkristalle mit hoher Koerzitiv-Feldstärke gemäß WO95/03061 z.B. Beispiel 2 oder 3 und WO98/44895 z.B. Beispiel 1C; Kaolin sowie mit $SiO_2$ modifiziertes Kaolin gemäß WO 94/17588.

Als Feuchthaltemittel können bevorzugt eingesetzt werden Glycerin, Butylenglycol, Propylenglycol und Gemische davon.

Ein weiterer Zusatz für das erfindungsgemäße Kosmetikum ist eine Wirkstoffzubereitung mit hohem Radikalschutzfaktor auf pflanzlicher Basis, wie in WO 99/66881 beschrieben.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, mineralische öle, Butylmyristat, Palmitinsäure usw.

Der genannte Extrakt der Rinde von Quebracho blanco wird nach der Extraktion enzymatische hydrolysiert und enthält wenigstens 90 Gew- % Proanthocyanidin-Oligomere und höchstens 10 Gew-% Gallussäure. Zusammen mit einem durch Extraktion gewonnenen Seidenraupenextrakt, der das Peptid Cecropine, Aminosäuren und ein Vitamingemisch enthält, und beide in einem nichtionischen, kationischen oder anionischen Hydro-Gel oder Gemisch von Hydro-Gelen, stellt einen bevorzugten Bestandteile der erfindungsgemäßen Formulierung dar, gegebenenfalls in Mikrokapseln vorliegend. Das Extraktgemisch ist aus der WO99/66881 bekannt und wird in der vorliegenden Erfindung z.B. als Wirkstoffkomplex gemäß Beispiel 1 oder 2 der WO99/66881 eingesetzt.

Es können weiterhin Antioxidationsmittel hinzugesetzt werden, wie Folsäure und deren Derivate, Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Äpfelsäure; stilbene und deren Derivate; sowie Granatapfelextrakte.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie $TiO_2$, $SiO_2$, ZnO, $Fe_2O_3$, $ZrO_2$, MnO, $Al_2O_3$, die auch im Gemisch eingesetzt werden können.

Besonders bevorzugt als anorganische Pigmente sind agglomerierte Substrate von $TiO_2$ und/oder ZnO, die einen Gehalt an sphärischen und porösen $SiO_2$-Teilchen aufweisen, wobei die $SiO_2$-Teilchen eine Teilchengröße im Bereich von 0,05 µm bis 1,5 µm haben, und neben den $SiO_2$-Teilchen andere anorganische teilchenförmige Stoffe mit sphärischer Struktur vorliegen, wobei die sphärischen $SiO_2$-Teilchen mit den anderen anorganischen Stoffen definierte Agglomerate mit einer Teilchengröße im Bereich von 0,06 µm bis 5 µm bilden.

Bevorzugt einzusetzende Enzyme sind solche eines kosmetischen Basiskomplexes, bestehend aus einer wäßrige Gelgrundlage und darin enthalten ein verkapselter Extrakt einer wäßrigen Extraktion der Ananas-Frucht und der Rückstand einer wäßrigen Extraktion von Joghurt, wobei die Extraktionen jeweils im Temperaturbereich von 10 bis 30 °C erfolgten und wobei das Verhältnis Ananas-Extrakt zu Joghurtextraktrückstand im Bereich von 20:80 bis 80:20 liegt und der Komplex einen Anteil des Enzyms Bromelin im Bereich von 0,1 bis 1 Gew-% aufweist.

Kosmetische Zusammensetzungen mit der erfindungsgemäßen Wirkstoffzubereitung können als O/W- oder W/O-Emulsionen vorliegen. Geeignete Emulgatoren für O/W-Emulsionen sind beispielsweise Anlagerungsprodukte von 2-30 Mol Ethylenoxid an lineare $C_8$-$C_{22}$-Fettalkohole, an $C_{12}$-$C_{22}$-Fettsäuren und an $C_8$-$C_{15}$-Alkylphenole; $C_{12}$-$C_{22}$-Fettsäuremono- und -diester von Anlagerungsprodukten von 1-30 Mol Ethylenoxid an Glycerin.

Geeignete Emulgatoren für W/O-Emulsionen sind beispielsweise Anlagerungsprodukte von 2-15 Mol Ethylenoxid an Ricinusöl; Ester von $C_{12}$-$C_{22}$-Fettsäuren und Glycerin, Polyglycerin, Pentaerythrit, Zuckeralkohole (z.B. Sorbit), Polyglucoside (z.B.

Cellulose); Polyalkylenglycole; Wollwachsalkohole; Copolymere von Polysiloxan-Polyalkylpolyether.

Die für die Erfindung eingesetzten öle können übliche kosmetische öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche öle; oder Gemische zweier oder mehrerer davon.

Besonders geeignet öle sind beispielsweise Hydrogenated Polyisobuten, Polyisopren, Squalane, Tridecyltrimellitat, Trimethylpropan-triisostearat, Isodecylcitrat, Neopentylglycoldiheptanoat, PPG-15-stearylether sowie pflanzliche öle, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon.

Die erfindungsgemäße kosmetische Zusammensetzung kann auch als Gel vorliegen. Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Der Zusatz von Pigmenten, Pigmentgemischen oder Pulver mit pigmentartiger Wirkung zu der erfindungsgemäßen Zubereitung ist möglich. Zu derartigen Pigmenten gehören auch solche mit Perlglanz-Effekt, oder solche wie Eisenoxide, natürliche Aluminiumsilicate wie Ocker, Titanoxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff.

Die Erfindung betrifft auch die Verwendung der oben beschriebenen kosmetischen Vitamin-A-haltigen Zubereitung als langandauerndes stimulans mit Anti-Falten-Wirkung und radikalfangender Wirkung.

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Reinigungsmilch, Make up's, Lippenstiften, Körperpuder, Augenkosmetik, Haarmasken, Haarspülungen, Haarshampoos, Duschgelen, Duschölen, Badeölen. Die Herstellung derartiger Produkte erfolgt auf eine Weise, wie sie dem Fachmann auf diesem Gebiet bekannt ist.

Es wurden Vergleichsversuche durchgeführt mit den Einzelbestandteilen der verschiedenen Anwendungsformen der Retinylderivate, wie in Anspruch 1 a) bis d) aufgeführt, und zwar der Kombination der Komponenten a), b) und c) und der Kombination der Komponenten a), b), c) und d) bei jeweils gleicher Konzentration der Retinylderivate wie im Beispiel 1 aufgeführt. Dabei wurde der Radikalschutzfaktor nach dem Auftragen der Zubereitungen auf Hauthomogenat in bestimmten Abständen gemessen.

Der Radikalschutzfaktor (RPF) bestimmt die Aktivität zur Bindung freier Radikale durch ein Antioxidationsmittel gegenüber einer Testsubstanz. Diese Testsubstanz besteht aus einem sehr reaktionsfähigen, halbstabilen Radikal, das mit allen bekannten Antioxidationsmitteln reagiert. Die Messung des RPF erfolgt in der Weise, daß die Signalamplitude des Testradikals durch Elektronenspinresonanz (ESR/EPR) vor und nach dem Vermischen mit dem zu bestimmenden Antioxidationsmittel (hier: Retinol bzw. Retinylderivat bzw. weitere antioxidativ wirkende Zusatzstoffe) gemessen und daraus der RPF berechnet wird.

Das genaue Meßverfahren ist beschrieben von Herrling, Groth, Fuchs und Zastrow in Conference Materials "Modern Challenges To The Cosmetic Formulation" 5.5.-7-5.97, Düsseldorf, S: 150-155, Verlag f. chem Ind. 1997. Dabei wird, ausgehend von der bekannten Konzentration des Testradikals oder der Anzahl von dessen freien Radikalen (Radikale pro ml) eine Signalamplitude $S_1$ mittels eines ESR-Spektrometers gemessen. Das Testradikal ist ebenso wie das Antioxidationsmittel (hier : Retinylderivat s.o.) in einer Wasser/Alkohollösung gelöst. Dann wird die Signalamplitude $S_2$ des Anti-oxidationsmittels gemessen. Die normalisierte Differenz zwischen den beiden Signalamplituden ist der Reduktionsfaktor RF.

$$RF = (S_1 - S_2) / S_1$$

Das Ergebnis der Testradikalreduzierung RC x RF wird zu der Menge der Produkteingabe PI (mg/ml) normalisiert. Der Radikalschutzfaktor wird nach der folgenden Gleichung berechnet

$$RPF = \frac{RC \text{ [Radikale/ml] x RF}}{PI \text{ [mg/ml]}}$$

Das Ergebnis ist RPF = N x $10^{14}$ [Radikale pro mg], wobei N eine positive reale Zahl ist. Die Wiedergabe des RPF erfolgt normalerweise ohne den Zusatz "x $10^{14}$", so daß sich eine einfache glatte Zahl ergibt.

Ein RPF <15 bedeutet keinen bedeutsamen Schutz gegenüber freien Radikalen mehr.

Für die Kombination a) bis c) ergab sich nach 4 Stunden ein RPF von 25, und für die Kombination a) bis d) nach 4 stunden ein RPF von 67. Nach 7 Stunden lag der RPF der Kombination a) bis d) bei 20, und der RPF der Kombination a) bis c) bei 9. Das zeigt eine bedeutsame Verlängerung der Wirkungszeit einer Kombination verschiedener Anwendungsformen des Retinols bzw. seiner Derivate auf der Haut durch Zusatz eines speziellen Pflanzenextraktes. Ähnliche Ergebnisse wie mit dem Prunusextrakt wurde mit einem Gemisch von Prunus- und Acerola-Extrakt erzielt. Ein solches Gemisch ist beispielsweise erhältlich von Greentech S.A., St. Beauzire, Frankreich unter der Bezeichnung ROSAMINE.

Die Erfindung soll nachstehend durch Beispiele näher erläutert werden. Alle Angaben erfolgen in Gewichtsprozent, sofern nichts anderes angegeben ist.

Beispiel 1 **Lotion**

a) Gehalt der Vitamin-A-Wirkstoffe:

Wirkstoff A :    0,5 % Retinylpalmitat
Wirkstoff B :    5,0 % Retinylpalmitat verkapselt in Liposomen (Phoslipon®)
Wirkstoff C :    1,0 % Mikrokapseln mit 1 Gew-% reines Retinol
Wirkstoff D :    3,0 % Extrakt der spanischen Cherry-Kirsche (Prunus cerasus) mit 0,015 % Retinylpalmitat

b) Zusammensetzung der Lotion nach Phasen

| Phase A | |
|---|---|
| Wasser | ad 100 |
| Glycerine | 2 |
| **Phase B** | |
| Cetyl Alcohol | 5 |
| Cetearyl Alcohol | 5 |
| PEG 40 Stearate | 1 |
| **Phase C** | |
| Vitamin E | 0,5 |
| Wirkstoff A, B, C und D | siehe oben |
| Parfüm | 0,5 |
| Konservierungsmittel | 0,1 |

c) Arbeitsweise

Phase A und B wurden separat gemischt und auf 75 °C erwärmt. Unter Rühren wurde die Phase B in die Phase A eingebracht und 15 Min homogenisiert. Danach wurde unter Rühren das Gemisch auf ca. 32 °C abgekühlt und die Phase C hinzugegeben, wobei dies bei langsamem Rühren bis zur Erreichung der Homogenität erfolgte.

Beispiel 2 **Antifaltencreme**

a) Gehalt der Vitamin-A-Wirkstoffe:

Wirkstoff A : 0,01 % Retinylpalmitat
Wirkstoff B : 10,0 % Retinylpalmitat verkapselt in Liposomen
Wirkstoff C : 0,1 % Mikrokapseln mit 1 Gew-% reines Retinol
Wirkstoff D : 10,0 % Extrakt der spanischen Cherry-Kirsche (Prunus cerasus) im Gemisch mit Acerola-Extrakt mit zusammen 0,02 % Retinylpalmitat

b) Zusammensetzung der Lotion nach Phasen:

| Phase A | |
|---|---|
| Wasser | ad 100 |
| Glycerine | 3 |
| Crosspolymer | 0,2 |
| **Phase B** | |
| Steareth 21 | 1,8 |
| Steareth 2 | 1,1 |
| Cetyl Alcohol | 2,5 |
| **Phase C** | |
| TEA | 0,2 |
| **Phase D** | |
| silicone | 3 |
| Konservierungsmittel | 0,8 |
| Parfümöl | 0,5 |
| **Phase E** | |
| Wirkstoff A, B, C und D | siehe oben |

c) Arbeitsweise

Phase A und B werden separat gemischt und auf 75 °C erwärmt. Unter Rühren wird die Phase B in die Phase A eingebracht und 15 Min homogenisiert. Danach wird unter Rühren das Gemisch auf ca. 32 °C abgekühlt und nacheinander die Phasen C, D und E hinzugegeben, wobei dies bei langsamem Rühren bis zur Erreichung der Homogenität erfolgt.

Beispiele 3-6

Es werden Antifaltencremes mit der Zusammensetzung der Lotion gemäß Beispiel 2b) mit gleicher Arbeitsweise hergestellt. Der Gehalt der Vitamin A-Wirkstoffe ist wie folgt (in Gew-%, bezogen auf die Gesamtzubereitung):

Tabelle 1

| Wirkstoff | Bsp. 3 | Bsp. 4 | Bsp. 5 | Bsp. 6 |
|---|---|---|---|---|
| A | | | | |
| Retinylascorbat | 0,8 | - | 0,6 | 0,8 |
| Retinylpalmitat | - | 0,1 | 0,05 | - |
| B | | | | |
| Retinylascorbat | - | - | 6,0 | - |
| Retinylpalmitat | 8,0 | 1,1 | - | 8,0 |
| C | | | | |
| Retinol | 2,4 | 4,2 | 0,08 | - |
| D | | | | |
| Quebracho-Extr. | 0,3 | - | - | 0,3 |
| Ananas-Extrakt | - | 0,9 | - | - |
| Seidenraupenextr. | - | - | 3,3 | - |

Dabei ist Beispiel 6 ein Vergleichsbeispiel zu dem Beispiel 3 ohne Gehalt an Wirkstoff C (verkapseltes Retinol). Retinylderivate von Wirkstoff D sind im Beispiel 1 Retinol, in Beispiel 2 , 3 und 6 Retinylpalmitat, in Beispiel 4 Retinylascorbat und in Beispiel 5 Retinol + Retinylpalmitat.

Beispiel 7

Es wurden Tests durchgeführt zum Nachweis der Antifaltenwirkung. Der Test wurde bei 21 Probanden/Probandinnen im Alter von 42 bis 58 Jahren durchgeführt. Das Mikrorelief von Abschnitten der Gesichtshaut (Augenpartie, Mundwinkel, Nasenpartie) wurde mittels einer Silikonmasse abgenommen, die Masse ausgehärtet und das erhaltene Negativrelief elektrooptisch vermessen in Bezug auf Höhe und Anzahl der Falten. Unmittelbar nach der Abnahme des Mikroreliefs erfolgte das erste Auftragen einer Creme im Gesicht der Probanden und danach täglich zweimal eine Wiederholung des Auftragens.

Gruppe 1:    12 Probanden erhielten die Creme von Beispiel 3;
Gruppe 2:    6 Probanden erhielten die Creme von Beispiel 6;
Gruppe 3:    3 Probanden erhielten eine Creme, die allein aus der Basisformulierung ohne Wirkstoffe bestand (Placebo).

Kontrollmessungen durch Abnehmen des Mikroreliefs der gleichen Hautpartien der einzelnen Probanden wurden am 14. und 28. Tag nach der ersten Messung vorgenommen. In dieser Zeit waren alle Probanden keinen besonderen Belastungen durch Sonneneinstrahlung unterworfen. Die ermittelten Werte sind statistische Mittelwerte über einen ausgewählten Bereich des Mikroreliefs.

Tabelle 2

| | Anzahl der Probanden | | |
|---|---|---|---|
| | Gruppe 1 | Gruppe 2 | Gruppe 3 |
| Verbesserung der Faltentiefe nach 14 Tagen | | | |
| - um 10-30 % | 9 | 4 | 1 |
| - um 30-50 % | 2 | - | - |
| Verbesserung der Faltentiefe nach 28 Tagen | | | |
| - um 30-50 % | 3 | 3 | - |
| - um 50-60 % | 6 | 1 | - |
| - um 60-70 % | 3 | - | - |

Die Gruppe 1 zeigt eine sehr gute Reduzierung der Faltentiefe nach 28 Tagen bei 75 % der probanden. Gegenüber der Gruppe 2, die ebenfalls eine Kombination von verschiedenen Vitamin A-Derivaten aufgetragen erhielt, zeigt die erfindungsgemäße Zusammensetzung gemäß Beispiel 3 noch einmal eine signifikante Verbesserung, die nicht ohne weiteres aus dem erhöhten Retinolgehalt gegenüber Beispiel 6 zu erwarten war, sondern eher auf einen noch nicht geklärten Synergismus hinweist.

**Patentansprüche**

1.   Kosmetische Vitamin-A-haltige Zubereitung, **gekennzeichnet durch**

a) 0,001 bis 1,0 Gew-% eines Retinylderivates, bezogen auf das Gewicht der kosmetischen Zubereitung, und ausgewählt unter Retinylpalmitat, Retinylascorbat und Gemischen davon; und

b) 0,01 bis 10 Gew-% eines Retinylderivates, ausgewählt unter Retinylpalmitat, Retinylascorbat und Gemischen davon und verkapselt in phospholipidhaltigen Liposomen und mit einer Teilchengröße der Liposomen im Bereich von 150 bis 800 nm, wobei der Gehalt an Retinylderivat auf das Gewicht der Liposomen bezogen ist; und

c) 0,01 bis 5 Gew-% Mikrokapseln mit reinem Retinol, wobei die Teilchengröße der Kapseln von 40 bis 100 nm beträgt, und die Kapseln reines Retinol im Bereich von 0,5 bis 3 Gew-% enthalten, bezogen auf das Gewicht der gefüllten Kapseln; und

d) 0,1 bis 20 Gew-%, bezogen auf das Gewicht der kosmetischen Zubereitung, eines Extraktes ausgewählt unter den wäßrigen Fruchtextrakten Kirsch-Extrakt (Prunus cerasus), Acerola-Extrakt (Malpighia punicifolia), Ananas-Extrakt, hydrolysiertem Rindenextrakt von Quebracho blanco, Seidenraupenextrakt und Gemischen dieser Extrakte, mit einem Gehalt des Extraktes von wenigstens einer Retinolverbindung, ausgewählt unter Retinol, Retinylpalmitat, Retinylascorbat und Gemischen davon, im Bereich von 0,01 bis 5 Gew-%, wobei diese Angabe auf das Trockengewicht des Extraktes bezogen ist; und

e) einem Rest bis 100 Gew-% an kosmetische Hilfsstoffen, Trägerstoffen und Gemischen davon.

2.   Zubereitung nach Anspruch 1, **dadurch gekennzeichnet, daß** der Fruchtextrakt ein Gemisch eines Kirsch-Extraktes mit einem Acerola-Extrakt ist.

3.   Zubereitung nach Anspruch 1, **dadurch gekennzeichnet, daß** die Kirsche eine spanische Cherry-Kirsche ist.

4.   Zubereitung nach Anspruch 2, **dadurch gekennzeichnet, daß** das Fruchtextraktgemisch in Kapseln mit einer Teilchengröße von 30 bis 100 µm verkapselt ist.

5.   Zubereitung nach Anspruch 4, **dadurch gekennzeichnet, daß** das in den Mikrokapseln enthaltene Retinylpalmitat im Gemisch vorliegt zusammen mit wenigstens einem weiteren Vitamin, ausgewählt unter einem Vitamin E-Derivat, Vitamin C, Vitamin F und Gemischen davon, und Carotinoiden.

6.   Zubereitung nach Anspruch 1, **dadurch gekennzeichnet, daß** das in Liposomen verkapselte Retinylderivat Retinylpalmitat ist.

7.   Zubereitung nach einem der Ansprüche 1 bis 6, **dadurch gekennzeichnet, daß** sie weitere Wirkstoffe enthält, ausgewählt unter Lichtschutzmitteln, Radikalfängern, Enzymen, Wirkstoffextrakten pflanzlicher Stoffe, Sauerstofftragenden Fluorcarbonen, magnetischen Einkristallen mit hoher Koerzitiv-Feldstärke, mit $SiO_2$ modifiziertem Kaolin und Gemischen davon.

8.   Zubereitung nach Anspruch 1, **dadurch gekennzeichnet, daß** sie zusätzlich einen kosmetischen Basiskomplex enthält, bestehend aus einer wäßrige Gelgrundlage und darin enthalten ein verkapselter Extrakt einer wäßrigen Extraktion der Ananas-Frucht und der Rückstand einer wäßrigen Extraktion von Joghurt, wobei die Extraktionen jeweils im Temperaturbereich von 10 bis 30 °C erfolgten und wobei das Verhältnis Ananas-Extrakt zu Joghurtextraktrückstand im Bereich von 20:80 bis 80:20 liegt und der Komplex einen Anteil des Enzyms Bromelin im Bereich von 0,1 bis 1 Gew-% aufweist.

9. Verwendung einer kosmetischen Vitamin-A-haltigen Zubereitung nach einem der Ansprüche 1 bis 8 als langandauerndes Stimulans mit Anti-Falten-Wirkung und radikalfangender Wirkung.

**Claims**

1. Cosmetic preparation containing vitamin A which comprises

   a) 0.001 to 1.0 percent by weight of a retinyl derivative, calculated with reference to the weight of the cosmetic preparation and selected from among retinyl palmitate, retinyl ascorbate and mixtures thereof; and
   b) 0.01 to 10 percent by weight of a retinyl derivative, selected from among retinyl palmitate, retinyl ascorbate and mixtures thereof and encapsulated in phospholipid-containing liposomes with a particle size of the liposomes in the range of 150 to 800 nm, the content of retinyl derivatives being calculated with reference to the weight of the liposomes; and
   c) 0.01 to 5 percent by weight of micro-capsules with pure retinol the particle size of the capsules being in the range of 40 to 100 nm and which contain pure retinol in the range of 0.5 to 3 percent by weight, calculated with reference to the weight of the filled capsules; and
   d) 0.1 to 20 percent by weight, calculated with reference to the weight of the cosmetic preparation, of an extract, selected from among the aqueous fruit extracts cherry extract *(Prunus cerasus)*, acerola extract *(Malpighia punicifolia)*, pineapple extract, hydrolized bark extract of *Quebracho blanco*, silkworm extract and mixtures of these extracts, with the content of the extract of at least one retinol compound, selected from among retinol, retinyl palmitate, retinyl ascorbate and mixtures thereof, being in the range of 0.01 to 5 percent by weight, this figure being calculated with reference to the dry weight of the extract; and
   e) a rest, up to 100 percent by weight, of cosmetic auxiliaries, carrier substances and mixtures thereof.

2. Preparation according to claim 1 wherein the fruit extract is a mixture of a cherry extract with an acerola extract.

3. Preparation according to claim 1 wherein the cherry is a Spanish cherry.

4. Preparation according to claim 2 wherein the fruit extract mixture is encapsulated in capsules with a particle size of 30 to 100 $\mu$m.

5. Preparation according to claim 4 wherein the retinyl palmitate which is contained in the micro-capsules is provided as a mixture together with at least another vitamin, selected from among a vitamin E derivative, vitamin C, vitamin F and mixtures thereof, and carotenoides.

6. Preparation according to claim 1 wherein the retinyl derivative that is encapsulated in liposomes is retinyl palmitate.

7. Preparation according to one of the claims 1 to 6 which comprises other active ingredients, selected from among sun screens, scavengers, enzymes, active ingredient extracts of plant-based substances, oxygen-carrying fluorocarbons, magnetic single crystals with a high coercive force, kaolin modified with $SiO_2$ and mixtures thereof.

8. Preparation according to claim 1 which comprises in addition a cosmetic base complex consisting of an aqueous gel base and contained therein an encapsulated extract of an aqueous extraction of the pineapple fruit and the residue of an aqueous extraction of yoghurt the extractions in each case having been made in the temperature range of 10 to 30 ° C, the ratio pineapple extract to yoghurt extract residue being in the range of 20:80 to 80:20 and the complex having a percentage of the enzyme Bromelin in the range of 0.1 to 1 percent by weight.

9. Use of a cosmetic preparation containing vitamin A according to one of the claims 1 to 8 as a long-term stimulant with an anti-wrinkle effect and scavenging properties.

**Revendications**

1. Préparation cosmétique contenant de la vitamine A, **caractérisée par**

   a) 0,001 à 1,0 % en poids d'un dérivé de rétinyle sur le poids de la préparation cosmétique, choisi parmi le palmitate de rétinyle, l'ascorbate de rétinyle et leurs mélanges ; et

b) 0,01 à 10 % en poids d'un dérivé de rétinyle, choisi parmi le palmitate de rétinyle, l'ascorbate de rétinyle et leurs mélanges, et encapsulé dans des liposomes phospholipidiques avec une taille de particules des liposomes de 150 à 800 nm, la teneur en dérivé de rétinyle étant rapportée sur le poids des liposomes ; et

c) 0,01 à 5 % en poids de microcapsules avec du rétinol pur, la taille de particules des capsules étant de 40 à 100 nm, et le rétinol pur étant compris de 0,5 à 3 % en poids, en fonction du poids des capsules remplies ; et

d) 0,1 à 20 % en poids, sur le poids de la préparation cosmétique, d'un extrait choisi parmi les extraits de fruit aqueux extrait de cerise (*Prunus cerasus*), extrait d'acérola (*Malpighia punicifolia*), extrait d'ananas, extrait d'écorce hydrolysée de *Quebracho blanco*, extrait de ver à soie et mélange de ces extraits, avec une concentration de l'extrait d'au moins un groupement rétinol, choisi parmi le rétinol, le palmitate de rétinyle, l'ascorbate de rétinyle et leurs mélanges, de 0,01 à 5 % en poids, cette valeur étant rapportée au poids sec de l'extrait ; et

e) un résidu jusqu'à 100 % en poids d'additifs cosmétiques, de substances porteuses et de leurs mélangés.

2. Préparation selon la revendication 1, **caractérisée en ce que** l'extrait de fruit est un mélange d'un extrait de cerise et d'un extrait d'acérola.

3. Préparation selon la revendication 1, **caractérisé en ce que** les cerises sont des griottes.

4. Préparation selon la revendication 2, **caractérisée en ce que** le mélange d'extraits de fruit est encapsulé dans une capsule avec une taille de particules de 30 à 100 μm.

5. Préparation selon la revendication 4, **caractérisée en ce que** le palmitate de rétinyle compris dans les microcapsules est présent avec au moins une autre vitamine, choisie parmi un dérivé de la vitamine E, la vitamine C, la vitamine F et leurs mélanges, et des caroténoïdes.

6. Préparation selon la revendication 1, **caractérisée en ce que** le dérivé de rétinyle encapsulé dans les liposomes est du palmitate de rétinyle.

7. Préparation selon l'une des revendications 1 à 6, **caractérisée en ce qu'**elle contient d'autres substances actives, choisies parmi des agents de protection solaire, des piégeurs de radicaux, des enzymes, des extraits de substances actives de substances végétales, des fluorocarbures porteurs d'oxygène, des monocristaux magnétiques ayant des champs coercitifs élevés, du kaolin modifié avec du SiO$_2$ et leurs mélanges.

8. Préparation selon la revendication 1, **caractérisée en ce qu'**elle contient en outre un complexe cosmétique de base, constitué d'une base de gel aqueuse et contenant un extrait encapsulé d'une extraction aqueuse d'ananas et du résidu d'une extraction aqueuse de yaourt, les extractions étant réalisées à une température de 10 à 30°C et le ratio de l'extrait d'ananas sur le résidu d'extrait de yaourt étant de 20/80 à 80/20 et le complexe présentant une part en enzyme broméline de 0,1 à 1 % en poids.

9. Utilisation d'une préparation cosmétique contenant de la vitamine A selon l'une des revendications 1 à 8 comme stimulant à long terme ayant une action anti-rides et une action de piégeur de radicaux libres.